# EUROPEAN PATENT SPECIFICATION

(11) **EP 4 049 638 B1**
(45) Date of publication and mention of the grant of the patent: **08.01.2025**
(21) Application number: 22162039.6
(22) Date of filing: 24.09.2015
(51) Int. Cl.: A61F 13/00, A61F 13/02

(54) **DEVICES FOR WOUND CLOSURE**
VORRICHTUNGEN ZUM WUNDVERSCHLUSS
DISPOSITIFS DE FERMETURE DE PLAIES

(43) Date of publication of application: 31.08.2022
(62) Divisional of application: 20167273.0
(73) Proprietor: Ethicon LLC, 00969 Guaynabo (PR)
(72) Inventor: QUINTERO, Julian A., SOMERVILLE, 08876 (US)
(74) Representative: Carpmaels & Ransford LLP

(56) References cited:
- US-A1- 2004 220 505
- US-A1- 2006 058 721

## Description

### Field of the Invention

This invention is concerned with devices for wound closure, particularly for skin closure of wounds formed by lacerations or surgical incisions.

### Related Art

Wound closure tapes and topical adhesives provide an alternative to wound closure by staples and sutures. Among the advantages of using wound closure tapes or topical adhesives, and their combinations include less tissue trauma, improved cosmetic outcomes, and less pain compared with staples and sutures when treating skin closures.

US 2002/0193721 discloses a wound closure grip-like tape apparatus and methods of its use. Reference to FIG. 10 of US 2002/0193721 and the corresponding text of this publication shows how the grip tape is used to secure the wound of FIG. 9. In particular, paragraph 43 of the publication describes three techniques of wound closure reproduced in part as follows: "The first exemplary means of sealing the wound is to place WCGT ("Wound Closure Grid Tape") **100** on one side of the wound **90** with a slight bend along the axis between numbers **92** and **93** such that the other side **91** is tilted above the skin and thus will not adhere. With side **90** pressed in place and with the appropriate pressure applied, the WCGT is tugged with one hand while the other hand (or the thumb of the hand if being self-applied or with one hand) pushes the skin on the other side **91** into position, and then the WCGT is lowered into place. This handling can be done by grasping the WCGT at the edges where denigration of the adhesive is not as critical, or alternately by leaving the backing attached to the secondary side **91** while the first side is pressed into place **90.** The second exemplary means to apply is to curve the WCGT upwards, or hold it in a curved position upwards along the longer axis shown, and position it starting at one end of the wound **92.** Then, as previously, the wound is pressed together and the WCGT is pressed into place gradually along the length of the wound, going from one end **92** to the other **93.** For small wounds, a third approach is to hold the WCGT by the edges and close the wound via pressures outside of the expected WCGT area, and then press the WCGT in place all at once."

In the forgoing description, denigration of the wound-contacting adhesive is a recognized problem since holding at least some of the underside of WCGT 100 is required particularly when side 90 of the wound is drawn toward side 91 of the wound. Thus, from the forgoing description, it appears that wound closure tape only to has a single release liner.

US 2008/0302487 A1 describes a dispensing device configured to operate with an adhesive backed mesh and backing film for tissue bonding. The device prevents or eliminates distortion of the mesh prior to application to the wound sites and includes means for reducing or eliminating binding during use. The dispensing device is configured to operate in a "forward" mode (substrate to which mesh is applied passes beneath applicator after mesh is applied) to provide essentially an unobstructed view of the wound site during use. The backing film is a single strip that protects denigration of adhesive and self-adherence of the coiled adhesive-backed mesh.

US2005/0182443A is directed to a tissue bonding article which includes a flexible material, an adhesive substance applied over at least a portion of a bottom side of the flexible material, and a polymerizable adhesive composition permeated throughout at least a portion of the flexible material. Although not specifically shown in the figures, a suitable backing or release material may also be used to cover the adhesive substances applied to the bottom side of the flexible material. Such backing materials are well known in the art for covering adhesives and can include, for example, paper, or plastic.

There continues to be a need for improved devices and systems that use surgical tapes, topical adhesives and their combinations such as those provided by this invention.

### SUMMARY OF THE INVENTION

The invention is defined by the appended claims.

Advantages of the invention include:
1) Minimization of adhesive transfer from the product to the user's fingers/gloves, therefore maximizing grip to tissue.
2) Due to the presence of release liner frames, ease of repositioning the wound closure strip once deployed on approximated wound edges.
3) Pressure sensitive adhesive is present on only the side that is intended to be in contact with tissue. So there is no accidental adherence of objects to the wound closure strip side not in contact with tissue.
4) Manipulation of the wound closure strip is stable during tissue approximation as the release liner frames provide sufficient rigidity.
5) Easy removal of the release liner central section with respect to the frames of the release liner.

### BRIEF DESCRIPTION OF THE DRAWINGS

Figs. 1a-1f depict a wound closure device comprising a three-part release liner in accordance with an embodiment of the invention.
Figs. 1a'-1f' depict a wound closure device comprising a three-part release liner in accordance with an embodiment of the invention.
Figs. 2a-2f depict a wound closure device comprising a three-part release liner in accordance with an embodiment of the invention.
Figs. 3a-3f depict a wound closure device comprising a three-part release liner in accordance with an embodiment of the invention.
Figs. 4a-4f depict a wound closure device comprising a three-part release liner in accordance with an embodiment of the invention.
Figs. 5a-5f depict a wound closure device comprising a three-part release liner in accordance with an embodiment of the invention.
Figs. 6a-6f depict a wound closure device comprising a three-part release liner in accordance with an embodiment of the invention.
Figs. 7a-7f depict a wound closure device comprising a three-part release liner in accordance with an embodiment of the invention.
Fig. 8 depicts a first step for use of the device in accordance with the invention.
Fig. 9 depicts a second step for use of the device in accordance with the invention.
Fig. 10 depicts a third step for use of the device in accordance with the invention.
Fig. 11 depicts a fourth step for use of the device in accordance with the invention.

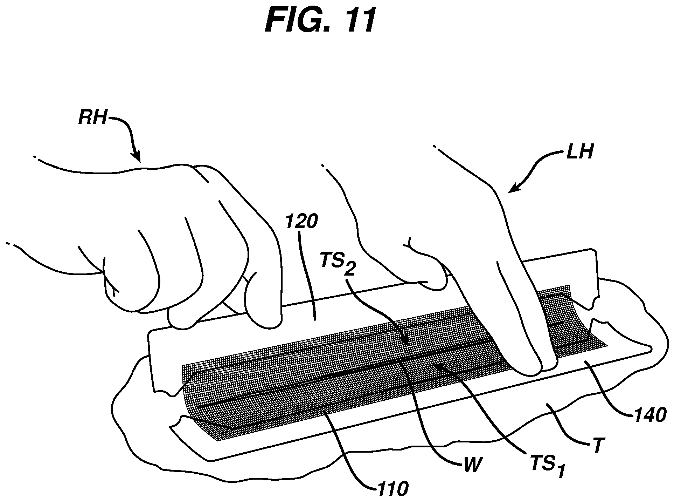

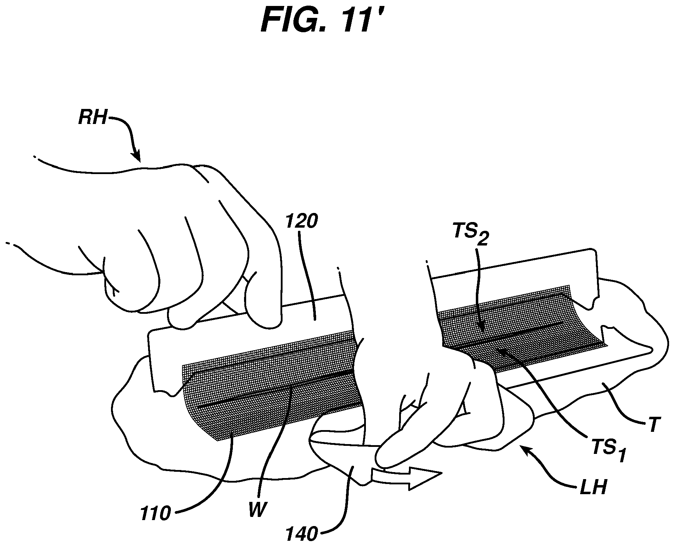

Fig. 11' depicts a modification to the fourth and fifth steps for use of the device in accordance with the invention.

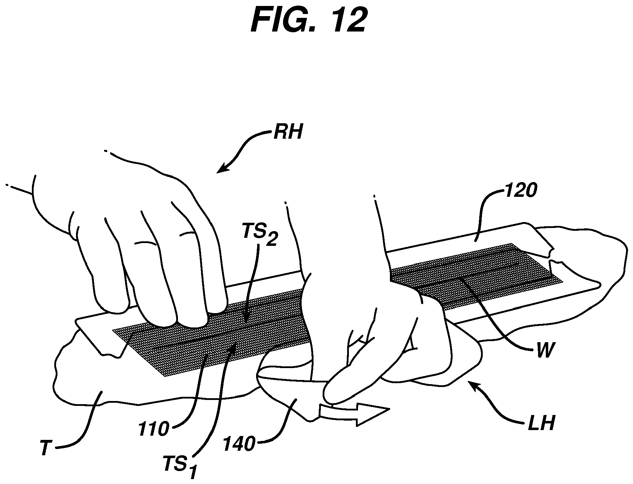

Fig. 12 depicts a fifth step for use of the device in accordance with the invention.

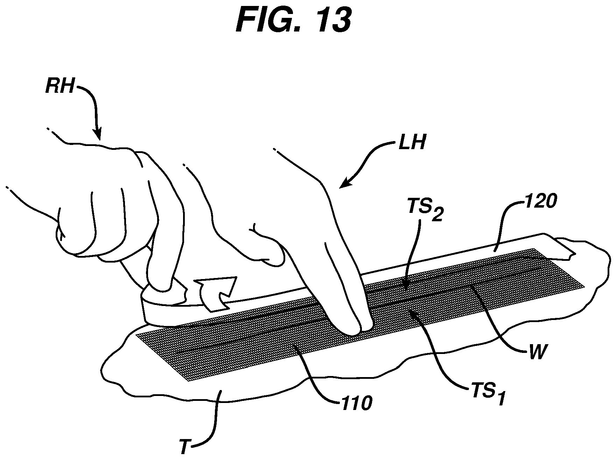

Fig. 13 depicts a sixth step for use of the device in accordance with the invention.

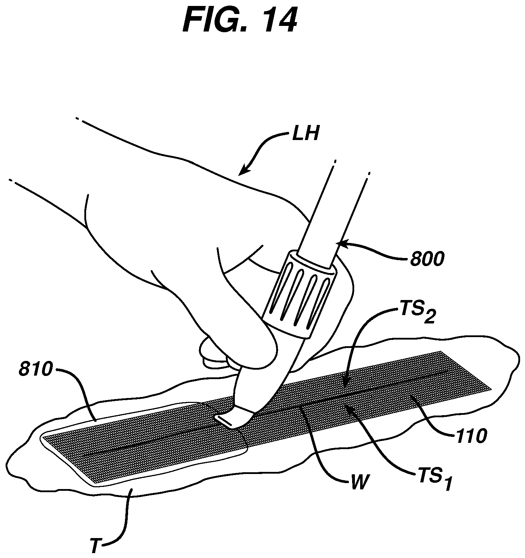

Fig. 14 depicts a further step for use of the device in accordance with the invention.

### DETAILED DESCRIPTION OF PREFERRED EMBODIMENTS OF THE INVENTION

A key aspect for the improved ease of use of wound closure devices of this invention lies in the three-part release liner assembly used in conjunction with a wound closure strip.

Wound closure strips suitable for use in this invention comprise any suitable strip that is adaptable to close a wound. Preferably, the wound closure strip is porous and will allow a flowable, polymerizable adhesive to permeate the strip and to allow adequate bonding of the strip to a tissue surface being bonded.

The wound closure strip comprises a wound facing side and a top side. The wound facing side further comprises an adhesive such as a pressure sensitive adhesive (PSA) applied over at least a portion of the wound facing side. The PSA is useful for initially approximating the wound. The wound closure strip is preferably porous. By "porous" is meant herein either that the bulk of the wound closure strip has pores, such that subsequently applied polymerizable adhesive composition is soaked up or absorbed by the bulk material, or that the bulk of the wound closure strip has voids (like a net or screen), such that the subsequently applied polymerizable adhesive composition passes directly through the bulk material, with or without being soaked up or absorbed by the bulk material. For example, in the case of textile materials, "porous" is generally used to mean that the applied adhesive composition permeates and passes through interstices between the fibers, but does not necessarily pass into and through the fibers themselves. Preferably the wound closure strip is a mesh.

Such porosity (or other properties such as hydrophobicity or hydrophilicity) will also allow a polymerization initiator or rate modifier to be loaded in or on the wound closure strip prior to use, to initiate the subsequently applied polymerizable adhesive composition. Such porosity will also preferably allow air and fluid to pass through the wound closure strip, either through pores per se, or through voids in the bulk material. Depending upon the degree of porosity and/or the size of the openings, such porosity of the mesh or ability of air and fluid to permeate through the mesh may be tailored either to remain after a final composite material is formed, or to be absent therefrom. The wound closure strip is also preferably non-toxic, as it is intended to be used cover a wound, such as on biological tissues. As such, the wound closure strip should be biologically compatible with the desired substrate (such as tissue, skin, organ), and is preferably a material that is governmentally approved or generally regarded as safe for the desired purpose. By way of example, suitable wound closure strips are mesh materials and are disclosed in United States Patent Applications 2006/0009099 and 2005/0182443.

The wound closure strip may be a textile or mesh/web material. Suitable textile materials may be formed of either synthetic or natural materials. Such textile material may be formed of either woven or non-woven fabrics or materials. The wound closure strip may be, for example, any suitable polymeric film, plastic foam (including open celled foam), a woven fabric, knitted fabric, a non-woven fabric, or mixture thereof. In particular, suitable wound closure strips may thus be prepared, for example, from nylon, a polyolefin film, such as polyethylene, polypropylene, ethylene propylene copolymers, and ethylene butylene copolymers, polyurethanes, polyurethane foams, polystyrenes, plasticized polyvinylchlorides, polyesters, polyamides, polylactic acid, polyglycolic acid, polycaprolactone, copolymer mixtures of the above, and cotton. Suitable specific examples include, for example, nylon, polyethylene, polypropylene, ethylene propylene copolymers, ethylene butylene copolymers, polyurethane, polystyrene, plasticized polyvinylchloride, polyester, polyamide, cotton, polytetrafluoroethylene (PTFE), biovascular material, collagen, Gore-Tex^{®}, and DACRON^{®}.

The wound closure strip may be formed of a synthetic, semi-synthetic, or natural organic material. Thus, for example, the mesh may be formed of a synthetic or natural polymer material, but not from a material such as metal (such as silver, or steel) or glass or ceramic. The wound closure strip may be either biodegradable, or not biodegradable. The wound closure strip is preferably resistant to tearing.

The thickness of the wound closure strip may be from about 0.1 mm to about 80 mm. In another embodiment, the thickness of the wound closure strip is from about 0.5 mm to about 20 mm, preferably from about 0.7 mm to about 10 mm, most preferably from about 1 mm to about 5 mm.

The wound closure strip may be from about 2 cm to about 40 cm, preferably from about 10 to about 30 cm, most preferably 25 cm in length. The strip may be from 0.1 to about 8 cm, preferably from about 2 to 6 cm, more preferably about 4 cm in width.

The wound closure strip may be selected to be elastic or have some memory effect. In such embodiments, the elastic properties of the mesh may desirably provide a degree of pressure or stress at the application site, for example, to maintain wound edge approximation. Likewise, in embodiments where such additional degree of pressure or stress at the application site is not desired, the mesh may be selected to have less or no elasticity.

The wound closure strip may be either biodegradable, or not biodegradable. By "biodegradable" is meant that the mesh biodegrades over time *in vivo,* such that it does not require physical removal of the mesh after a set period of time. Thus, for example, a biodegradable mesh is one that, in the *in vivo* environment, will biodegrade over a period of from about one week to about five years. A nonbiodegradable material is one that does not biodegrade in an *in vivo* environment within about five years. Such a nonbiodegradable material thus would require physical removal of the wound closure strip at a desired time, rather than slowly deteriorating over time or may slough off naturally from the tissue.

The wound closure strip preferably includes one or more chemical materials located in or on it. For example, one or more chemical substances may be dispersed in or on the wound closure strip, such as being chemically bound, physically bound, absorbed, or adsorbed to it. Thus, for example, the wound closure strip preferably includes at least a polymerization initiator or rate modifier, and may optionally include one or more bioactive materials. As desired, the one or more chemical substances may be either immobilized in or on the wound closure strip, for example, so that it has a desired effect but is not detached from the wound closure strip during use.

For example, a polymerization initiator or rate modifier may be loaded in or on the wound closure strip so that the initiator or rate modifier provides the desired initiation or rate modification effect to a subsequently applied polymerizable adhesive composition. The polymerization initiator or rate modifier may be immobilized in or on the wound closure strip, so that the initiator or rate modifier does not become detached from the wound closure strip and its residues are dispersed in the resultant polymeric material. Alternatively, for example, the polymerization initiator or rate modifier may be initially attached to the wound closure strip, but only in such a manner that it becomes mobilized or solubilized by a subsequently applied polymerizable adhesive composition and dispersed in the resultant polymeric material.

If desired, a combination of chemical substances may also be provided in or on the wound closure strip, to provide multiple effects. For example, as described above, a first chemical species (such as a polymerization initiator or rate modifier) may be immobilized in or on the wound closure strip, while a second, different chemical species (such as a bioactive material) may be detachably attached to the wound closure strip. Other combinations of chemical species and resultant effects are also envisioned.

The chemical substance may be applied in a uniform manner to the wound closure strip, such that there is a substantially uniform concentration of the chemical substance across the wound closure strip. Alternatively, the chemical substance may be applied such that a concentration gradient exists across or through the wound closure strip. For example, a greater or smaller concentration of the chemical substance could exist at the center or edges of the wound closure strip, or a greater or smaller concentration of the chemical substance could be applied on one side of the wound closure strip as compared to an opposite side. Further, the chemical substance may be applied in a uniform manner to the wound closure strip, or it may be applied in a non-uniform random or patterned manner (such as lines, dots, or concentric circles). The chemical substances may also be on, beneath, or in the adhesive layer applied to the wound closure strip.

When present in or on the wound closure strip, the chemical substances (*i.e*., polymerization initiator, rate modifier, and/or bioactive materials, or other additives), may be incorporated in or on the wound closure strip in any suitable manner. For example, the chemical substance may be wound closure strip added to the wound closure strip by contacting the wound closure strip with a solution, or mixture, including the chemical substances. The chemical substance may be added to the wound closure strip, for example, by dipping, spraying, roll coating, gravure coating, brushing, or vapor deposition. Alternatively, the chemical substance may be incorporated into or onto the wound closure strip during manufacture of the wound closure strip, such as during molding, knitting/weaving, scouring, tenting, plaiting or other processing of the wound closure strip.

Other chemical substances that may be present in or on the wound closure strip include, but are not limited to, any suitable and preferably compatible additive that enhances performance of the composite structure. Such additional chemical substances may be bioactive or non-bioactive. Suitable other chemical substances thus include, but are not limited to, colorants (such as inks, dyes and pigments), scents, protective coatings that do not chemically detach, temperature sensitive agents, drugs, wound-healing agents, and anti-microbial agents.

The polymerization initiator or rate modifier loaded in or on the wound closure strip may provide a number of advantages for example, the tailoring of the setting or polymerization time of the applied polymerizable adhesive composition. For example, the type and/or concentration of initiator that is applied to the wound closure strip may be selected so as to provide faster or slower polymerization time. The concentration of polymerization initiator or rate modifier may be increased to provide a faster polymerization time, or may be decreased to provide a slower polymerization time.

Because the polymerization initiator or rate modifier is loaded directly in or on the wound closure strip, it is not necessary to mix the polymerizable adhesive composition with a polymerization initiator or rate modifier prior to application. This may allow a longer working time, where the polymerizable monomer composition may be more precisely and carefully applied over a longer period of time.

Such suitable initiators are known in the art and are described, for example, in U.S. Pat. Nos. 5,928,611 and 6,620,846 and U.S. Patent Application No. 2002/0037310. Quaternary ammonium chloride and bromide salts useful as polymerization initiators are particularly suitable. By way of example, quaternary ammonium salts such as domiphen bromide, butyrylcholine chloride, benzalkonium bromide, acetyl choline chloride, among others, may be used.

Benzalkonium or benzyltrialkyl ammonium halides such as benzyltrialkyl ammonium chloride may be used. When used, the benzalkonium halide may be benzalkonium halide in its unpurified state, which comprises a mixture of varying chain-length compounds, or it can be any suitable purified compound including those having a chain length of from about 12 to about 18 carbon atoms, including but not limited to C12, C13, C14, C15, C16, C17, and C18 compounds. By way of example, the initiator may be a quaternary ammonium chloride salt such as benzyltrialkyl ammonium chloride (BTAC).

Other initiators or accelerators may also be selected by one of ordinary skill in the art without undue experimentation. Such suitable initiators or accelerators may include, but are not limited to, detergent compositions; surfactants: *e.g*., nonionic surfactants such as polysorbate 20 (*e.g*., Tween 20^{™} from ICI Americas), polysorbate 80 (*e.g*., Tween 80^{™} from ICI Americas) and poloxamers, cationic surfactants such as tetrabutylammonium bromide, anionic surfactants such as sodium tetradecyl sulfate, and amphoteric or zwitterionic surfactants such as dodecyldimethyl(3-sulfopropyl)ammonium hydroxide, inner salt; amines, imines and amides, such as imidazole, arginine and povidine; phosphines, phosphites and phosphonium salts, such as triphenylphosphine and triethyl phosphite; alcohols such as ethylene glycol, methyl gallate; tannins; inorganic bases and salts, such as sodium bisulfite, calcium sulfate and sodium silicate; sulfur compounds such as thiourea and polysulfides; polymeric cyclic ethers such as monensin, nonactin, crown ethers, calixarenes and polymeric-epoxides; cyclic and acyclic carbonates, such as diethyl carbonate; phase transfer catalysts such as Aliquat 336; organometallics such as cobalt naphthenate and manganese acetylacetonate; and radical initiators or accelerators and radicals, such as di-t-butyl peroxide and azobisisobutyronitrile.

Mixtures of two or more, such as three, four, or more, initiators or accelerators may be used. A combination of multiple initiators or accelerators may be beneficial, for example, to tailor the initiator of the polymerizable monomer species. For example, where a blend of monomers is used, a blend of initiators may provide superior results to a single initiator. For example, the blend of initiators can provide one initiator that preferentially initiates one monomer, and a second initiator that preferentially initiates the other monomer, or can provide initiation rates to help ensure that both monomer species are initiated at equivalent, or desired non-equivalent, rates. In this manner, a blend of initiators can help minimize the amount of initiator necessary. Furthermore, a blend of initiators may enhance the polymerization reaction kinetics. The polymerization initiator, accelerator, rate-modifier, and/or crosslinking agent may be incorporated into the mesh using impregnation methods known in the art.

The three-part release liner assembly is a backing film comprising multiple, individually peelable sections, most preferably in three sections, each section may be of same dimensions, more preferably, the sections in the outer portion may be of identical or similar dimensions and shapes.

The material for the invention's release liner assembly may be any suitable backing or release material used to cover the adhesive substances applied to the wound facing side of the wound closure strip. Such backing materials are well known in the art for covering adhesives and can include, for example, paper, or plastic. By way of example, the release liner assembly may be a silicone treated material. Preferably, the release liner assembly is of a material that prevents or eliminates the wound closure strip from sticking to itself.

The wound closure strip and the release liner assembly may overlap 100% (*i.e.,* be coextensive), but preferably allowing a release liner exposed margin of 0.1 to 1.5 cm, more preferably about 1.0 cm of release liner margin. By "release liner margin" with respect to width, it is intended to mean that the release liner assembly has a width which is such that it exceeds the strip width so that there is a margin on the liner assembly on one or both sides which is exposed according to the aforementioned margin. In addition, or alternatively, the strip and the release liner assembly may overlap lengthwise allowing paper lining exposure sections of 0.2 cm to 8 cm, preferably of about 0.5 cm to 5 cm, and more preferred of about 1.5 cm. Again, by "release liner margin" with respect to length, it is intended to mean that the release liner assembly has a length which is such that it exceeds the strip length so that there is a margin on the liner assembly on one or both ends which is exposed according to the aforementioned margin.

The method of wound closure or tissue bonding herein disclosed includes a polymerizable adhesive composition applied over the wound closure strip after the wound closure strip is applied to a tissue or wound site. The polymerizable adhesive composition may comprise a polymerizable monomeric adhesive. In embodiments, the polymerizable adhesive composition comprises a polymerizable 1,1-disubstituted ethylene monomer formulation. In embodiments, the polymerizable adhesive composition comprises a cyanoacrylate formulation. In embodiments, synthetic polymerizable adhesive materials such as polyurethane, polyethylene glycol, acrylates, glutaraldehyde and biologically based adhesives may be used.

Suitable α-cyanoacrylate monomers which may be used, alone or in combination, include alkyl α-cyanoacrylates such as 2-octyl cyanoacrylate; dodecyl cyanoacrylate; 2-ethylhexyl cyanoacrylate; butyl cyanoacrylate such as n-butyl cyanoacrylate; ethyl cyanoacrylate; methyl cyanoacrylate or other α-cyanoacrylate monomers such as methoxyethyl cyanoacrylate; 2-ethoxyethyl cyanoacrylate; 3-methoxybutyl cyanoacrylate; 2-butoxyethyl cyanoacrylate; 2-isopropoxyethyl cyanoacrylate; and 1-methoxy-2-propyl cyanoacrylate. In embodiments, the monomers are ethyl, n-butyl, or 2-octyl α-cyanoacrylate. Other cyanoacrylate monomers which may be used include alkyl ester cyanoacrylates, such as those prepared by the Knoevenagel reaction of an alkyl cyanoacetate, or an alkyl ester cyanoacetate, with paraformaldehyde, subsequent thermal cracking of the resultant oligomer and distillation.

The wound closure device herein disclosed may be provided in a kit comprising additional components. The kit may comprise at least one mesh strip as herein described, and one or more containers of polymerizable adhesive composition. The different components or groups of components may be sterilized in separate containers before packaging the components or groups of components within a kit, and thereafter sterilizing the kit as disclosed in co-assigned U.S. Pregrant Patent Publication No. 2004/0120849.

The adhesive substance used in the mesh may, for example, be any suitable adhesive substance. Preferably, the adhesive substance is a medical grade adhesive, such as acrylic based pressure sensitive adhesives (PSAs), rubber based pressure sensitive adhesives, silicone pressure sensitive adhesives, or mixtures thereof. It is preferred that the adhesive substance be different from the polymerizable adhesive composition. Thus, for example, it is preferred that while the polymerizable adhesive composition can be, for example, a polymerizable monomeric adhesive composition, the adhesive substance is a material that is not a polymerizable adhesive composition, such as a pressure sensitive adhesive.

Suitable rubber based PSAs include, but are not limited to, those taught in U.S. Pat. No. 5,705,551 and in U.S. Pat. No. 4,080,348. Examples of polymeric rubber bases include one or more of styrene-isoprene-styrene polymers, styrene-olefin-styrene polymers including styrene-ethylene/propylene-styrene polymers, polyisobutylene, styrene-butadiene-styrene polymers, polyisoprene, polybutadiene, natural rubber, silicone rubber, acrylonitrile rubber, nitrile rubber, polyurethane rubber, polyisobutylene rubber, butyl rubber, halobutyl rubber including bromobutyl rubber, butadiene-acrylonitrile rubber, polychloroprene, and styrene-butadiene rubber.

A particularly useful rubber based adhesive is that which has a thermoplastic elastomeric component and a resin component. The thermoplastic elastomeric component contains about 55-85 parts of a simple A-B block copolymer wherein the A-blocks are derived from styrene homologs and the B-blocks are derived from isoprene, and about 15-45 parts of a linear or radical A-B-A block copolymer wherein the A-blocks are derived from styrene or styrene homologs and the B-blocks are derived from conjugated dienes or lower alkenes, the A-blocks in the A-B block copolymer constituting about 10-18 percent by weight of the A-B copolymer and the total A-B and A-B-A copolymers containing about 20 percent or less styrene. The resin component consists of essentially of tackifier resins for the elastomeric component. In general, any compatible conventional tackifier resin or mixture of such resins may be used. These include hydrocarbon resins, rosin and rosin derivatives, polyterpenes and other tackifiers. The adhesive substance may contain about 20-300 parts of the resin component per one hundred parts by weight of the thermoplastic elastomeric component. One such rubber based adhesive substance is commercially available from Ato Findley under the trade name HM3210.

Useful acrylic based PSAs include, but are not limited to, those taught in U.S. Pat. No. 5,947,917 and U.S. Pat. No. 5,164,444 (acrylic emulsion), U.S. Pat. No. 5,623,011 (tackified acrylic emulsion). It can also be radiation curable mixture of monomers with initiators and other ingredients such as those taught in U.S. Pat. No. 5,232,958 (UV cured acrylic) and U.S. Pat. No. 5,232,958 (EB cured) .

It is contemplated that any acrylic based polymer capable of forming an adhesive layer with sufficient tack to adhere to the wound closure strip, the backing film or to a substrate, and with acceptable adhesion to skin, may be used. In certain embodiments, the acrylic polymers for the pressure-sensitive adhesive layers include those formed from polymerization of at least one alkyl acrylate monomer or methacrylate, an unsaturated carboxylic acid and optionally a vinyl lactam. Examples of suitable alkyl acrylate or methacrylate esters include, but are not limited to, butyl acrylate, ethyl acrylate, 2-ethylhexyl acrylate, isooctyl acrylate, isononyl acrylate, isodecyl acrylate, methyl acrylate, methylbutyl acrylate, 4-methyl-2-pentyl acrylate, sec-butyl acrylate, ethyl methacrylate, isodecyl methacrylate, and methyl methacrylate, and mixtures thereof. Examples of suitable ethylenically unsaturated carboxylic acids include, but are not limited to, acrylic acid, methacrylic acid, fumaric acid, and itaconic acid, and mixtures thereof. A preferred ethylenically unsaturated carboxylic acid monomer is acrylic acid. Examples of suitable vinyl lactams include, but are not limited to, N-vinyl caprolactam, 1-vinyl-2-piperidone, 1-vinyl-5-methyl-2-pyrrolidone, and vinyl pyrrolidone, and mixtures thereof.

Useful silicone pressure sensitive adhesives include those commercially available from Dow Corning Corp., Medical Products and those available from General Electric. Examples of silicone adhesives available from Dow Corning include those sold under the trademarks BIO-PSA X7-3027, BIO-PSA X7-4919, BIO-PSA X7-2685, BIO-PSA X7-3122 and BIO-PSA X7-4502. Additional examples of silicone pressure sensitive adhesives are described in U.S. Pat. Nos. 4,591,622, 4,584,355, 4,585,836 and 4,655,767.

The adhesive substance may also include one or more tackifiers, plasticizers, antioxidants, cutting agents such as waxes, and surfactants. Other optional materials that may be added to the adhesive substance layer in minor amounts (typically less than about 25% by weight of the elastomeric phase) include pH controllers, medicaments, bactericides, growth factors, wound healing components such as collagen, antioxidants, deodorants, perfumes, antimicrobials and fungicides.

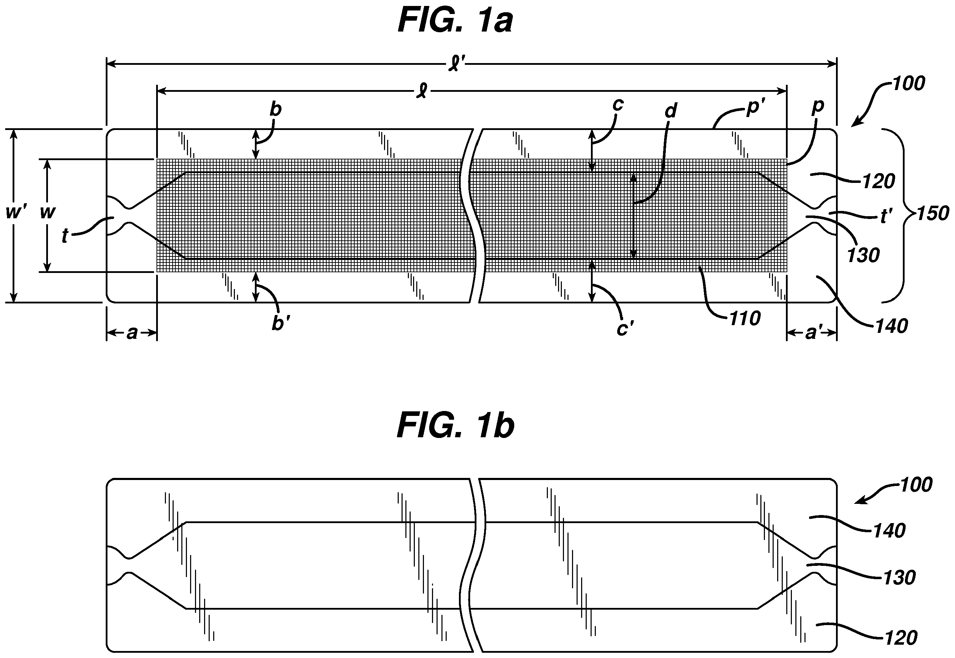

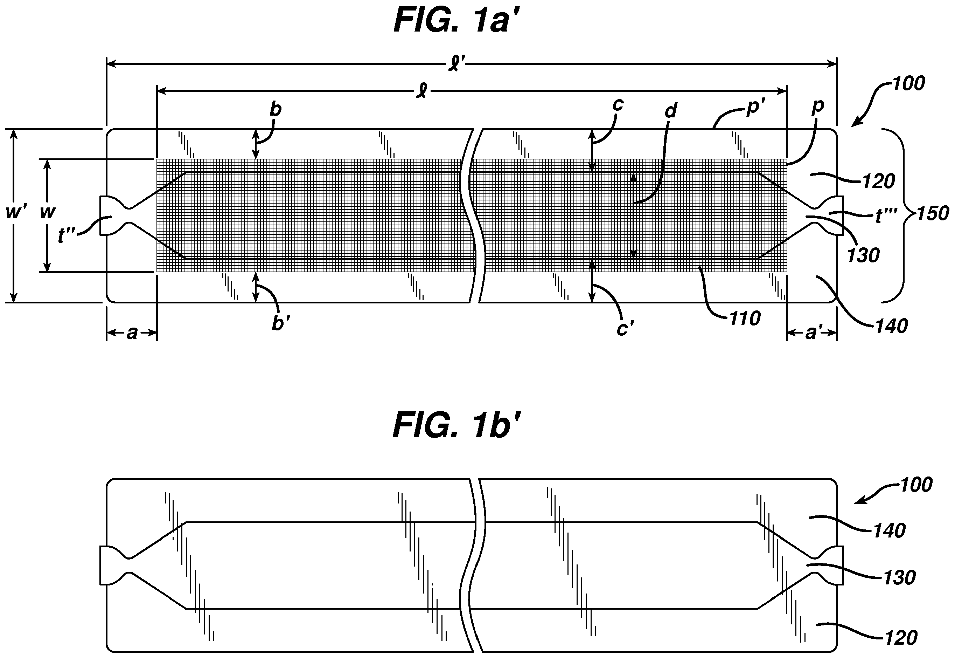

Figs. 1a-1f depict wound closure device 100 having an overall length l and overall width w and comprising a three-part release liner assembly 150 in accordance with one embodiment of the invention. In particular, Fig. 1a is a front view of device 100 comprising a mesh 110 having perimeter p and a release or backing assembly 150 having perimeter p' comprising first frame 120, central section 130 having pull tabs t and t', and second frame 140.

In certain embodiments, the release liner assembly 150 may be a release liner assembly. By "central" section, it is intended to mean that this section merely resides between the first frame 120 and second frame 140, rather than necessarily being centrally located. In a particular more limiting embodiment, the section 130 may be centrally located with respect to the first frame 120 and second frame 140. While two tabs t' and t" are shown, it will be appreciated that in certain embodiments only a single tab t' may be present at one end of the release assembly 150.

Distances a and a' represent the distances of the release liner assembly perimeter p' to mesh 110 perimeter p of the left hand and right hand sides of device 100, respectively. Distances b and b' represent the distances from the release liner assembly perimeter p' to mesh 110 perimeter p of the top and bottom sides of device 100, respectively. Distances c and c' represent the width of first frame 120 and second frame 140, respectively, and coinciding with the width of central section 130 at dimension d.

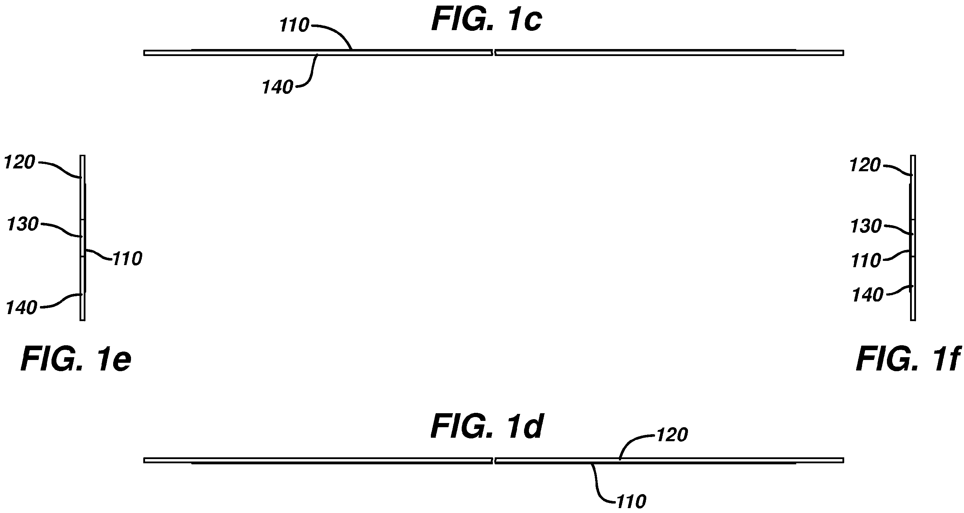

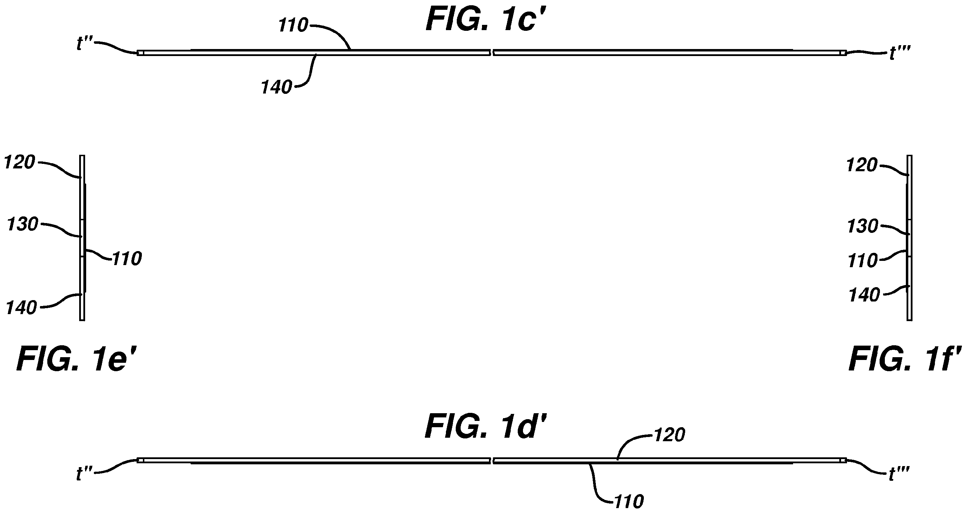

Fig. 1b is a rear view of the device. Figs. 1c and 1d are top and bottom views of device 100, respectively. Figs. 1e and 1f are left-side and right-side views, respectively of device 100.

Many embodiments are evident for device 100 depending on the specific dimensions one skilled in the art may select and adapt to treat wounds or incisions of various sizes and shapes.

For example, referring to Fig. 1a, it is envisioned that mesh 110 perimeter p can be coextensive with device 100 perimeter p', partially coextensive or not be coextensive as indicated by the following discussion.

Device 100 may be of any length l' and width w' that permits the user to effectively apply to close a wound or incision. For many applications, length l' will typically range from about 2 cm to about 40 cm, preferably from about 10 cm to 30 about cm, and most preferably about 25 cm. Width w' will typically range from about 1 cm to about 15 cm, preferably from about 2 to about 10 cm, and most preferably about 6 cm.

Wound closure strip 110 may be of any length l and width w that permits the user to effectively apply to close a wound or incision. For many applications, length l will typically range from about 2 cm to about 40 cm, preferably from about 10 cm to about 30 cm, and most preferably about 25 cm. Width w' will typically range from about 0.1 cm to about 8 cm, preferably from about 2 cm to about 6 cm, and most preferably about 4 cm.

Again referring to Fig. 1a, the distances a and a' may be the same or different and range from 0.0 cm to about 8 cm, preferably from about 0.5 cm to about 5 cm, and most preferably about 1.5 cm. It is within the foregoing ranges device 100 is conveniently handled.

In certain circumstances, for example (but not limited to) when the dimensions of a or a' is 0.0 cm, either one or both pull tabs, t or t', extend or protrude beyond the perimeter p', for example beyond one or both ends of the first frame 120 and second frame 140. Extended tabs t" and t‴ are shown in Figs. 1a' to 1d' (see below). In this way, the user is able to more easily remove center section 130 from device 100 since the edge of one or both tabs t", t‴ of the center section 130 protrude away from the corresponding edge of the first frame 120 and second frame 140 making each tab t", t‴ easier to grip for removal. Of course, in an embodiment in which only one pull tab t' ' is present, then only this tab may protrude beyond its corresponding one end of the first frame 120 and second frame 140.

Dimensions b and b' may be the same or different and range from 0.0 cm to about 2.5 cm, preferably from about 0.5 cm to about 1.5 cm, and most preferably about 1.0 cm. It is within the foregoing ranges that the margins of frames 120 and 140 and of wound closure strip 110 are defined. Dimensions b and b' provide sufficient margin for manipulation of wound closure strip 110 during the approximation of a wound in a manner that lessens the tendency for the adhesive (and other additives that may be present on or in wound closure strip, such as initiators) to become denigrated. While it is preferred that dimensions b and b' are the same, it is envisioned that there may be some applications on the human anatomy where it is desirable to have one of margins b and b' narrower or wider than the other.

Dimensions c and c' may be the same or different and range from 0.1 cm to about 3.0 cm, preferably from about 0.2 cm to about 2.0 cm, and most preferably about 1.5 cm. It is within the foregoing ranges that frames 120 and 140 provide sufficient rigidity for manipulation of wound closure strip 110 during the approximation of a wound. While it is preferred that dimensions c and c' are the same, it is envisioned that there may be some applications on the human anatomy where it is desirable to have one of frames 120 and 140 narrower or wider than the other.

Dimension d relates to the width of wound closure strip 110 that is suitable for the user to position and/or reposition wound closure strip 110 for the initial approximation of the separated topical tissue surfaces of a wound. Dimension d ranges from about 1.0 cm to about 8.0 cm, preferably from about 2.0 cm to about 6.0 cm, and most preferably about 3.0 cm.

For treatment of surgical wound incisions of about 10 cm to about 20 cm, a preferred embodiment of device 100 is for an overall length l' of about 25 cm and overall width w' of about 8 cm, wound closure strip of overall length l of about 22 cm and overall width w of about 4 cm with dimensions a and a' equal and about 1.5 cm, dimensions b and b' equal and about 1.0 cm, dimensions c and c' equal and about 1.5 cm, and dimension d about 3 cm. It would be appreciated by one of skill in the art that device 100 may be cut or trimmed to size for wounds within the above identified range. In practice, it is desirable to have wound closure strip 110 extend approximately at least 0.3 cm, preferably 1 cm from the longitudinal ends of the wound or incision site.

Figs. 1a'-1f' depict a wound closure device comprising a three-part release liner in accordance with another embodiment of the invention. This embodiment depicts a variation of the device shown in Figs. 1a - 1f wherein tabs t" and t‴ are shown to project or extend beyond the general perimeter p' of release liner assembly 150, for example beyond one or both ends of the first frame 120 and second frame 130. This embodiment is useful for more easily removing central section 130. For example, this is particularly advantageous if there is little or no margin between wound closure strip perimeter p and release liner assembly perimeter p', although it is advantageous even when a larger margin also exists. Of course, one example of such an instance would be when perimeters p and p' are coextensive with each other, except of course, for the protrusions of tabs t" and t‴. In general, with the protruding tabs t", t‴, the user is able to more easily remove center section 130 from device 100 since the edge of one or both tabs t", t‴ of the center section 130 protrude away from the corresponding edge of the first frame 120 and second frame 140 making each tab t", t‴ easier to grip for removal. Of course, in an embodiment in which only one pull tab t" is present, then only this tab may protrude beyond its corresponding one end of the first frame 120 and second frame 140.

Each tab may extend beyond a perimeter of the first frame and/or second frame in a direction substantially coincident with a longitudinal axis of the release assembly. The at least one tab may protrude beyond the perimeter of the first frame and/or second frame by an amount which is at least 0.05 cm, 0.1 cm, 0.2 cm, 0.3 cm, 0.4 cm, 0.5 cm, 1 cm, 1.5 cm, 2 cm, or 5 cm. The central section may be joined to the first frame and/or second frame by a frangible connection which is adapted to break upon pulling of the tab with respect to the first and/or second frame. Each tab may have a shape which is substantially one of the following: triangular, elliptical, square, rectangular, pentagonal, and hexagonal.

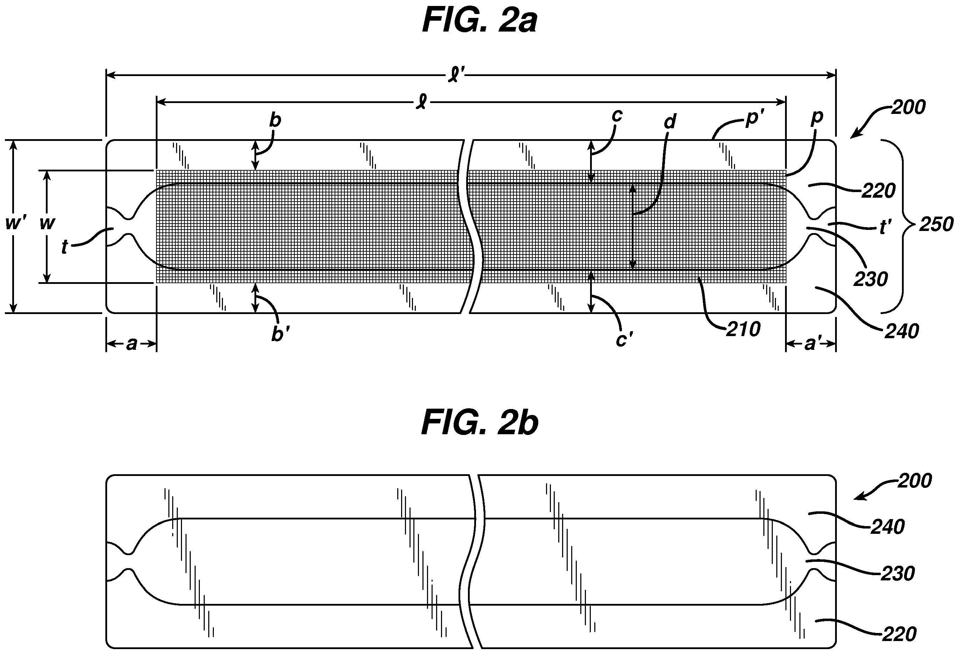

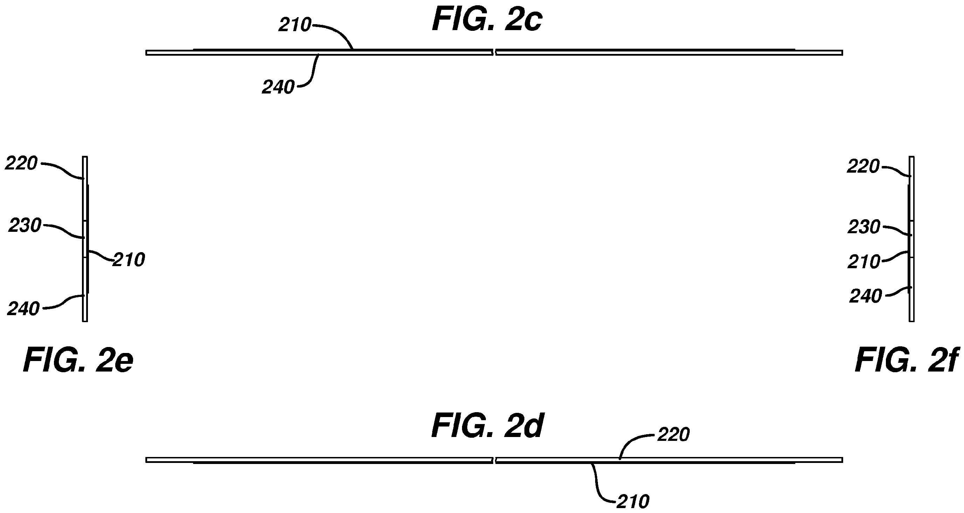

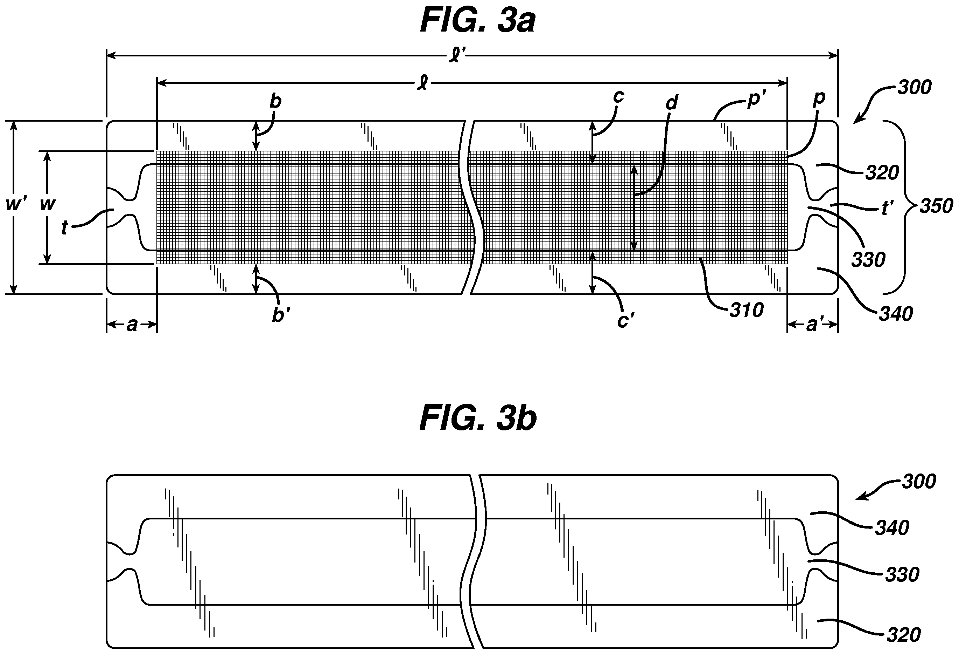

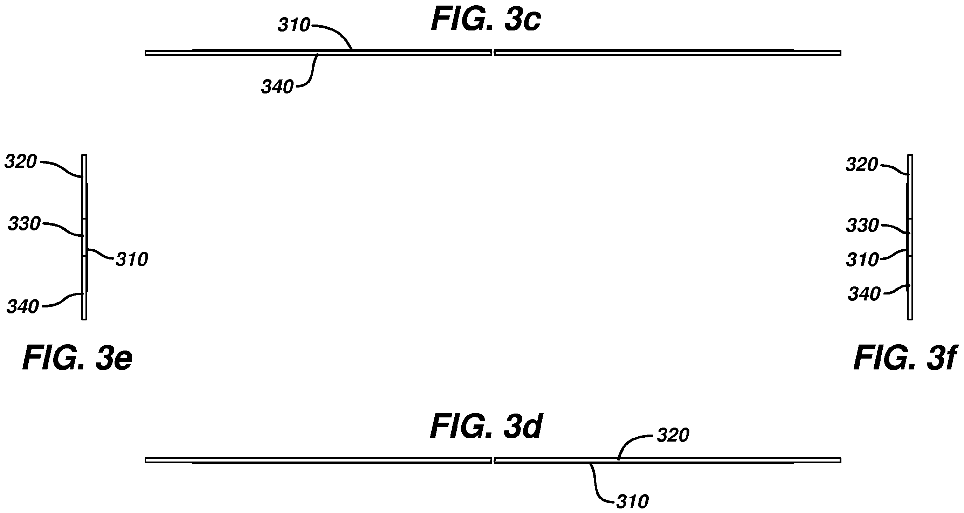

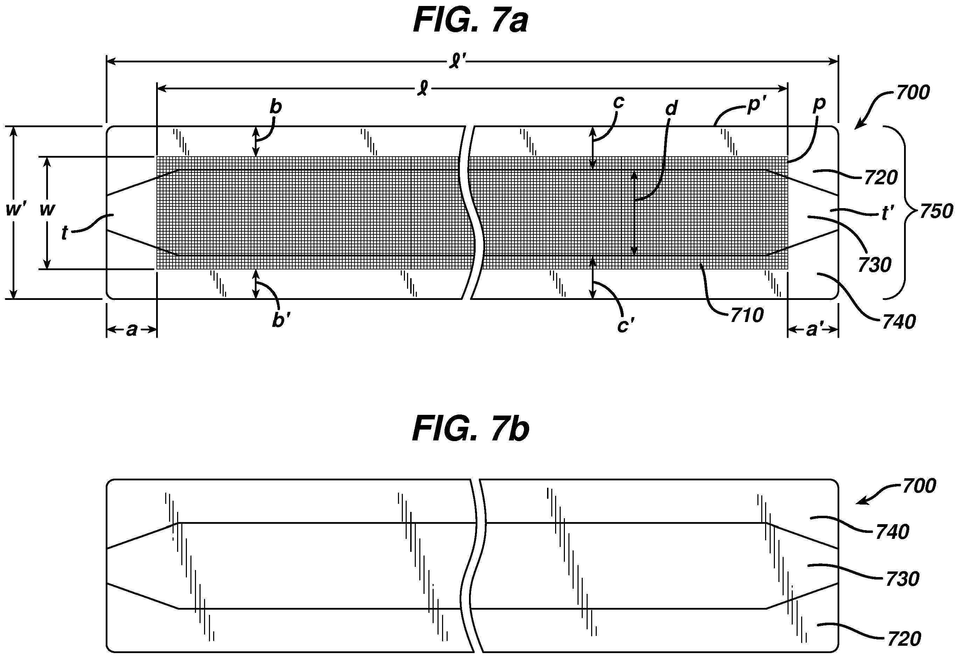

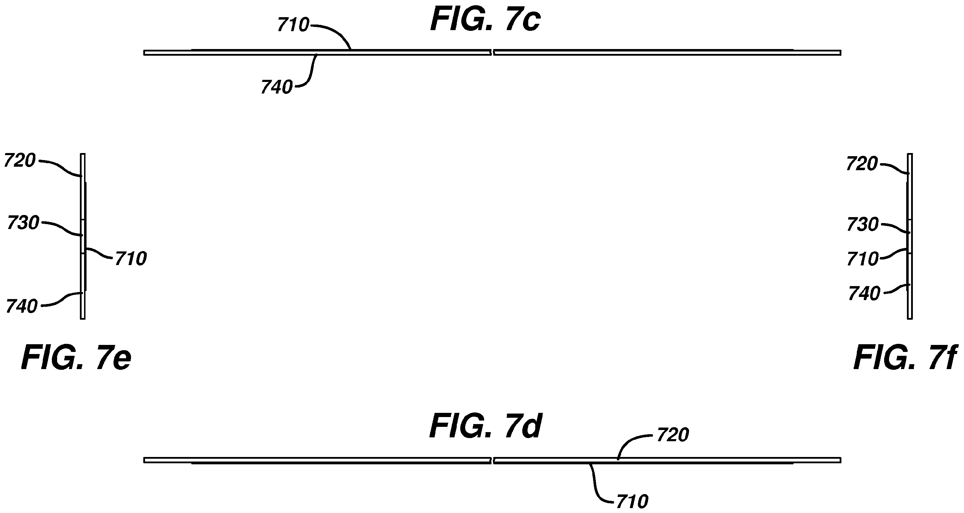

As a point of clarity, the device illustrations of Figs. 1a'-1f' to Figs. 7a-7f, inclusive, depict variations in the design of central section 130 of Figs. 1a-1f. As such, all previously defined parameters described for Figs. 1a-1f apply and have the same meaning. The numbering of the corresponding elements of the variously depicted embodiments for the devices follow the same numbering convention as in Figs. 1a-1f and only differ in that the numbering corresponds to series numbering specific to each set of illustrations (e.g., 200 series numbering for elements of Figs. 2a-2f, 300 series numbering for elements of Figs. 3a-3f, etc.) Therefore, further detailed description is not provided as one skilled in the art would appreciate and understand the commonality and defined meaning of the elements and dimensions described therein.

Figs. 2a-2f depict a wound closure device comprising a three-part release liner in accordance with another embodiment of the invention.

Figs. 3a-3f depict a wound closure device comprising a three-part release liner in accordance with a further embodiment of the invention.

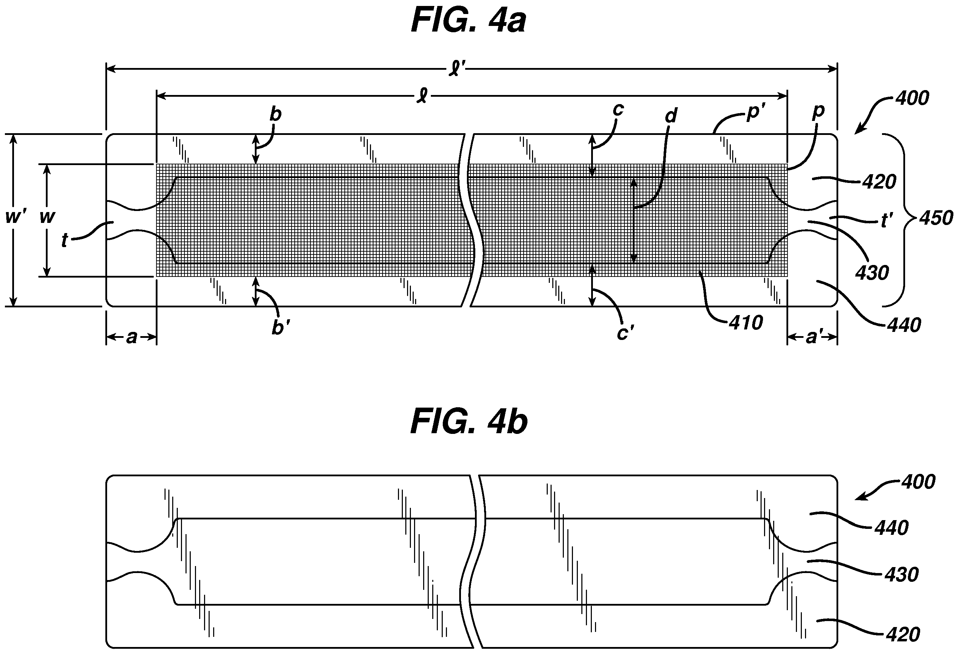

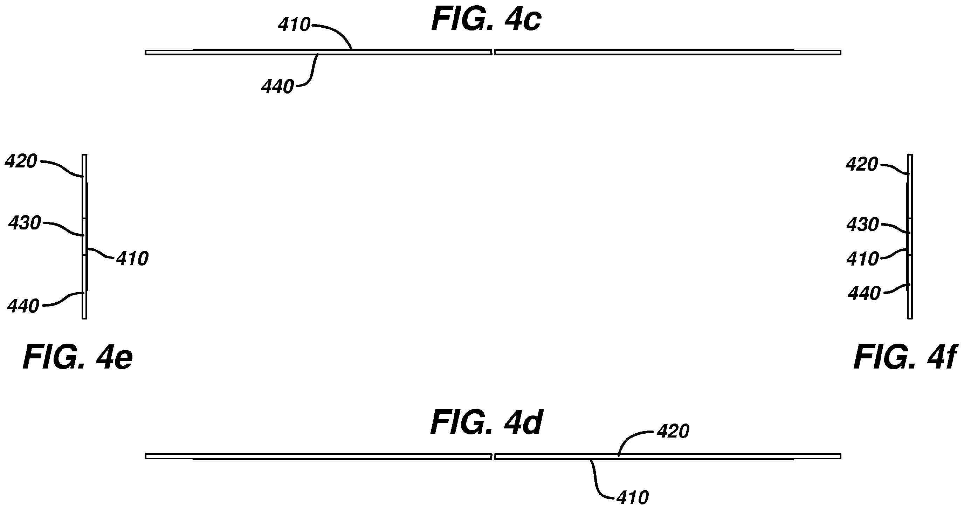

Figs. 4a-4f depict a wound closure device comprising a three-part release liner in accordance with another embodiment of the invention.

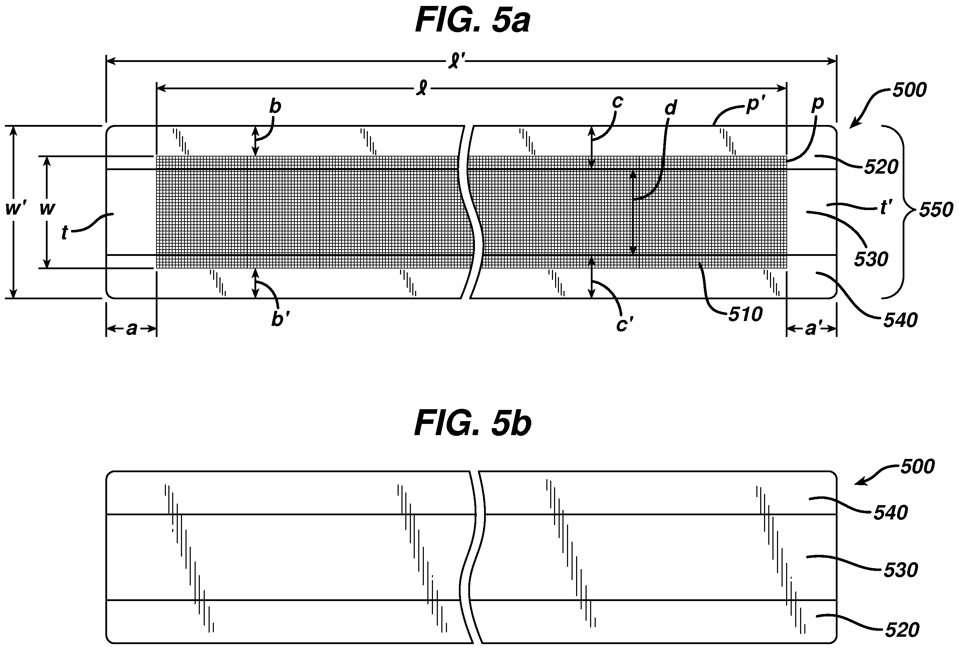

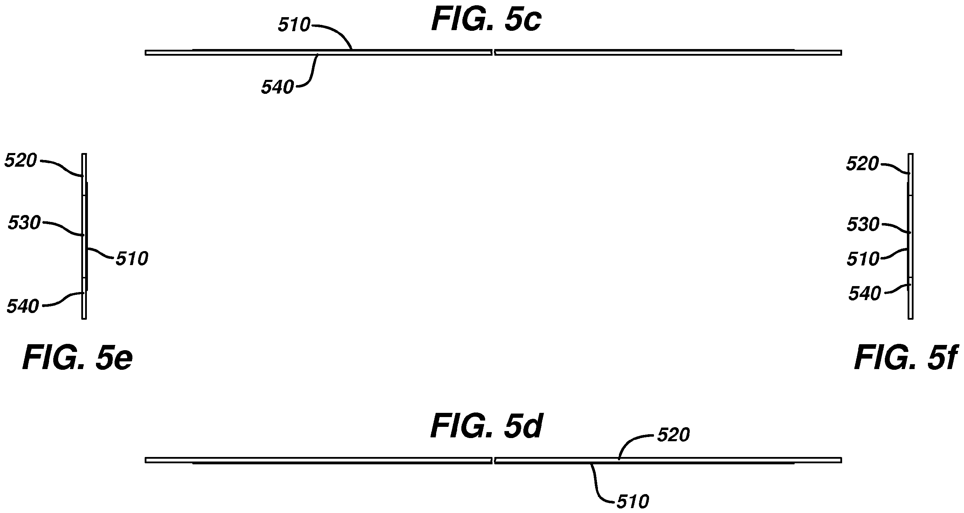

Figs. 5a-5f depict a wound closure device comprising a three-part release liner in accordance with a further embodiment of the invention.

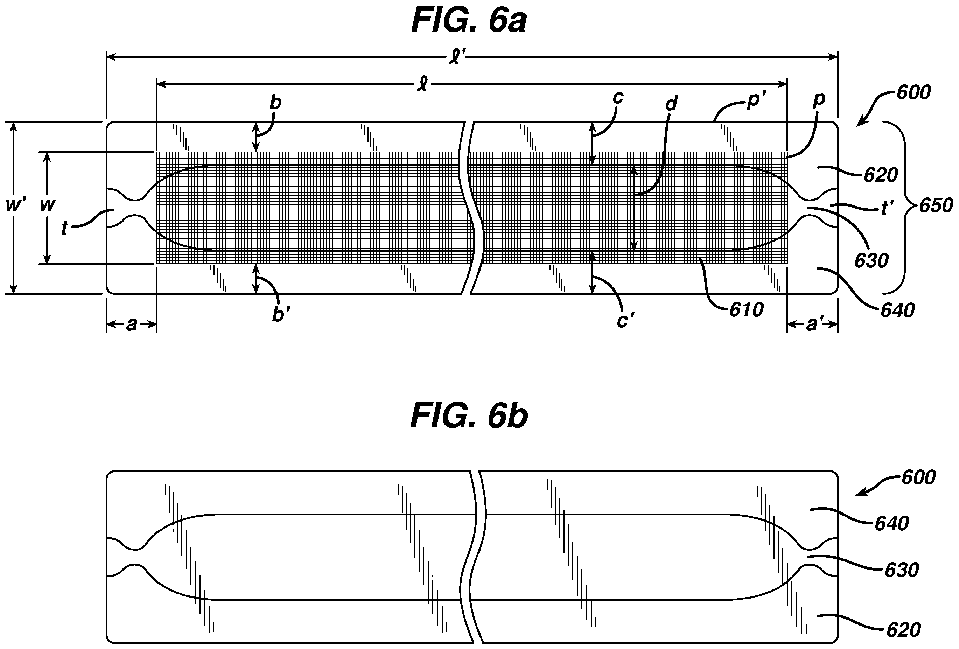

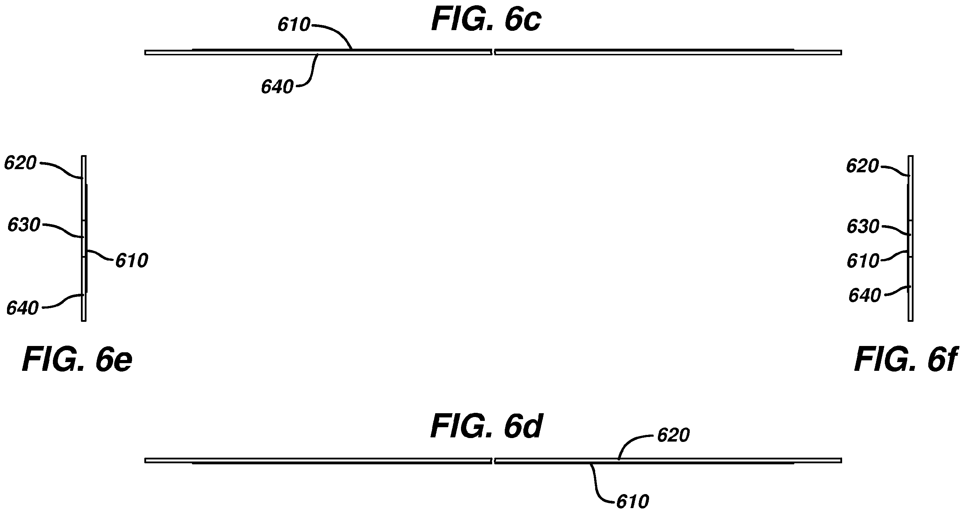

Figs. 6a-6f depict a wound closure device comprising a three-part release liner in accordance with another embodiment of the invention.

Figs. 7a-7f depict a wound closure device comprising a three-part release liner in accordance with a further embodiment of the invention.

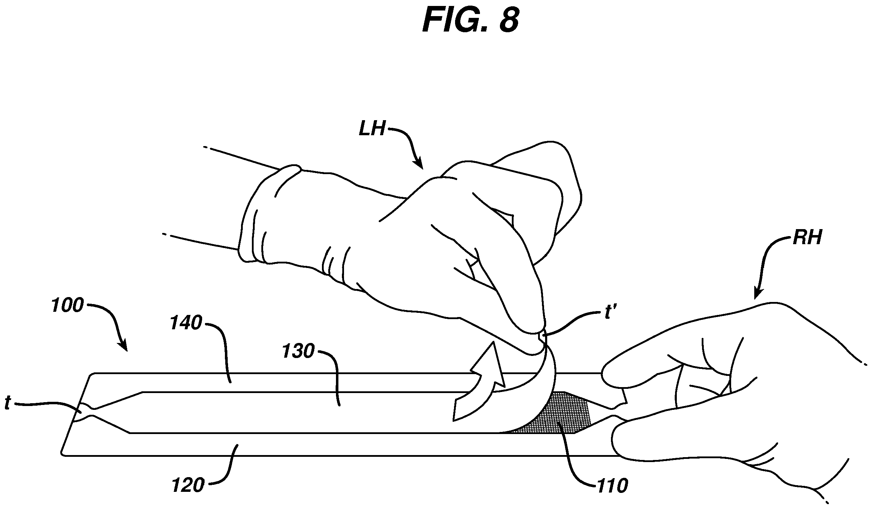

Figs. 8- 14 depict steps for the use of the device of this invention.

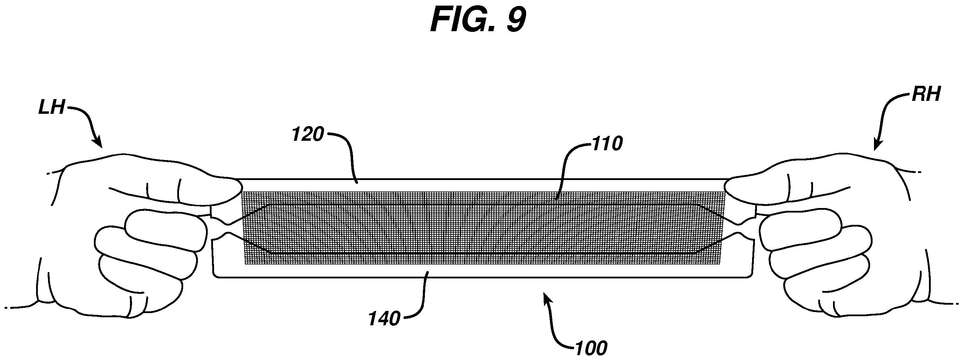

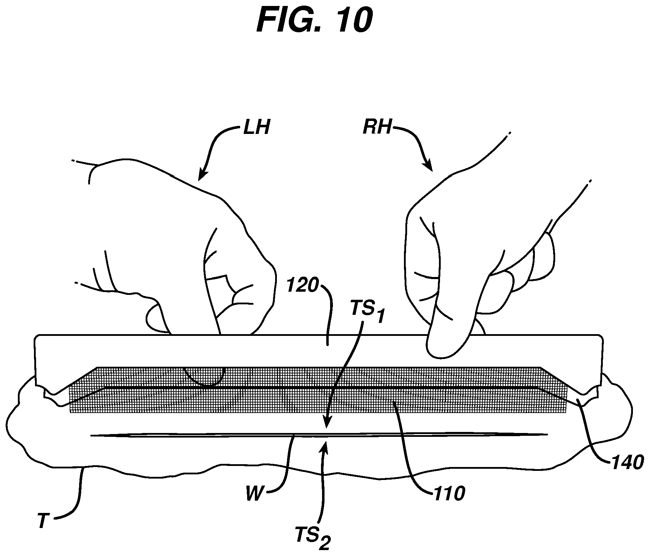

In particular, once a suitable wound has been identified for use of the invention's device and referring to Fig. 8, center section 130 of wound closure device 100 is removed by grasping and pulling either t' or t. Next, Fig. 9 illustrates wound closure device 100 being grasped at first frame 120. Subsequently, Fig. 10 shows the device being positioned axially along a side of wound W of tissue T and contacting a first separated topical tissue surface TS₁. Fig. 11 depicts an alternate view of tissue surface TS₁ on one side of wound W being contacted by the adhesive containing side of wound closure strip 110 with left hand LH of a user and initially being pulled to approximate a second separated topical tissue surface TS₂ of wound W to the first separated topical tissue surface TS₁ of wound W by pulling first frame 120 with right hand RH of the user. Figure 12 shows second frame 140 being removed from wound closure strip 110 by left hand LH of the user and Fig. 13 shows first frame 120 being removed from wound closure strip 110 by right hand RH of the user. Once the both frames 120 and 140 are removed, a suitable flowable, polymerizable adhesive may be applied from applicator 800 which permeates wound closure strip 100 to form film 810.

It would be understood by one of skill in the art that in the practice of this afore described method, once central section 130 of the release liner assembly is removed, the selection of whether the user chooses to grasp or remove the first frame 120 or second frame 140 is immaterial to the practice of this invention provided that the subsequent steps of the method used accomplishes closure of the wound.

Furthermore, as a modification to the above method, and referring to Fig. 11', prior to performing the approximation of topical tissue surface TS₁ to topical tissue surface TS₂, frame 140 may be removed and wound closure strip 110 may be further adhered to topical tissue surface TS₁. This variation to the technique is useful when additional gripping of TS₁ is desired or needed prior to completing the wound approximation.

Advantages of the methods, devices and systems of this invention include: clear visualization for placement of mesh 110 to approximate wound W by abutting the separate topical tissue surfaces of wound W; flexibility to reposition mesh 100 and adjust the approximation of wound W (i.e., the abutment of the separated topical tissue surfaces of the W) before mesh 110 is fully placed on the abutted topical tissue surfaces; ability to handle placement of mesh 110 without denigrating or minimizing denigration of the adhesive side of mesh 110; manipulation of mesh 110 is more stable during tissue approximation as frames 120 and 140 provide rigidity for mesh 110.

## Claims

1. A wound closure device comprising:
a wound closure strip having a wound facing side and a top side, the wound facing side comprising an adhesive applied over at least a portion of the wound facing side;
a release liner assembly detachably adhered to the strip by the adhesive, the release liner assembly being a backing film comprising a first frame, a central section, and a second frame which are individually peelable;
wherein the central section of the release liner assembly comprises at least one tab located at an edge of the release liner assembly;
and wherein the wound closure strip does not include a polymerization initiator or rate modifier.

2. The device of claim 1, wherein the wound closure strip comprises a mesh.

3. The device of claim 1, wherein the wound closure strip has an outer perimeter and the release liner assembly has an outer perimeter wherein at least a portion of the outer perimeter of the wound closure strip lies within the outer perimeter of the release liner assembly.

4. The device of claim 3, wherein the entirety of outer perimeter of the wound closure strip lies within the outer perimeter of the release liner assembly.

5. The device of claim 3, wherein the outer perimeter of the wound closure strip and the outer perimeter of the release liner assembly are coextensive.

6. The device of claim 5, wherein the wound closure strip further comprises a longitudinal axis and the release liner assembly further comprises a longitudinal axis and wherein a further portion of the central section of the release liner assembly extends beyond the outer perimeter of the wound closure strip along the wound closure strip's longitudinal axis.

7. The device of any one of the preceding claims, wherein the central section of the release liner assembly comprises at least two tabs located at opposing edges of the release liner assembly.

8. The device of any one of the preceding claims, wherein the central section is joined to the first frame and/or second frame by a frangible connection which is adapted to break upon pulling of the tab with respect to the first and/or second frame.

9. The device of any one of the preceding claims, wherein each tab has a shape which is substantially one of the following: triangular, elliptical, square, rectangular, pentagonal, and hexagonal.

## Patentansprüche

1. Wundverschlussvorrichtung, umfassend:
einen Wundverschlussstreifen mit einer wundzugewandten Seite und einer Oberseite, wobei die wundzugewandte Seite einen Klebstoff umfasst, der auf mindestens einen Teil der wundzugewandten Seite aufgetragen ist;
eine Trennfolienanordnung, die durch den Klebstoff lösbar an dem Streifen befestigt ist, wobei die Trennfolienanordnung eine Trägerfolie ist, die aus einem ersten Rahmen, einem Mittelabschnitt und einem zweiten Rahmen besteht, die einzeln abziehbar sind;
wobei der Mittelabschnitt der Trennfolienanordnung mindestens eine Lasche umfasst, die sich an einer Kante der Trennfolienanordnung befindet;
und wobei der Wundverschlussstreifen keinen Polymerisationsinitiator oder Geschwindigkeitsmodifikator einschließt.

2. Vorrichtung nach Anspruch 1, wobei der Wundverschlussstreifen ein Netz umfasst.

3. Vorrichtung nach Anspruch 1, wobei der Wundverschlussstreifen einen Außenumfang aufweist und die Trennfolienanordnung einen Außenumfang aufweist, wobei mindestens ein Teil des Außenumfangs des Wundverschlussstreifens innerhalb des Außenumfangs der Trennfolienanordnung liegt.

4. Vorrichtung nach Anspruch 3, wobei der gesamte Außenumfang des Wundverschlussstreifens innerhalb des Außenumfangs der Trennfolienanordnung liegt.

5. Vorrichtung nach Anspruch 3, wobei der Außenumfang des Wundverschlussstreifens und der Außenumfang der Trennfolienanordnung flächengleich sind.

6. Vorrichtung nach Anspruch 5, wobei der Wundverschlussstreifen ferner eine Längsachse umfasst und die Trennfolienanordnung ferner eine Längsachse umfasst und wobei ein weiterer Teil des Mittelabschnitts der Trennfolienanordnung über den Außenumfang des Wundverschlussstreifens entlang der Längsachse des Wundverschlussstreifens hinausragt.

7. Vorrichtung nach einem der vorstehenden Ansprüche, wobei der Mittelabschnitt der Trennfolienanordnung mindestens zwei Laschen umfasst, die sich an gegenüberliegenden Kanten der Trennfolienanordnung befinden.

8. Vorrichtung nach einem der vorstehenden Ansprüche, wobei der Mittelabschnitt mit dem ersten Rahmen und/oder dem zweiten Rahmen durch eine zerbrechliche Verbindung verbunden ist, die so ausgebildet ist, dass sie beim Ziehen der Lasche in Bezug auf den ersten und/oder zweiten Rahmen bricht.

9. Vorrichtung nach einem der vorstehenden Ansprüche, wobei jede Lasche im Wesentlichen eine der folgenden Formen aufweist: dreieckig, elliptisch, quadratisch, rechteckig, fünfeckig oder sechseckig.

## Revendications

1. Dispositif de fermeture de plaie comprenant :
une bande de fermeture de plaie ayant une face tournée vers la plaie et une face supérieure, la face tournée vers la plaie comprenant un adhésif appliqué sur au moins une partie de la face tournée vers la plaie ;
un ensemble doublure détachable collé à la bande par l'adhésif, l'ensemble doublure détachable étant un film support comprenant un premier cadre, une section centrale, et un second cadre qui sont individuellement pelables ;
dans lequel la section centrale de l'ensemble doublure détachable comprend au moins une languette située au niveau d'un bord de l'ensemble doublure détachable ;
et dans lequel la bande de fermeture de plaie ne comporte pas d'initiateur de polymérisation ou de modificateur de vitesse.

2. Dispositif selon la revendication 1, dans lequel la bande de fermeture de plaie comprend un maillage.

3. Dispositif selon la revendication 1, dans lequel la bande de fermeture de plaie a un périmètre externe et l'ensemble doublure détachable a un périmètre externe, dans lequel au moins une partie du périmètre externe de la bande de fermeture de plaie se trouve à l'intérieur du périmètre externe de l'ensemble doublure détachable.

4. Dispositif selon la revendication 3, dans lequel la totalité du périmètre externe de la bande de fermeture de plaie se trouve à l'intérieur du périmètre externe de l'ensemble doublure détachable.

5. Dispositif selon la revendication 3, dans lequel le périmètre externe de
la bande de fermeture de plaie et le périmètre externe de l'ensemble doublure détachable sont coextensifs.

6. Dispositif selon la revendication 5, dans lequel la bande de fermeture de plaie comprend en outre un axe longitudinal et l'ensemble doublure détachable comprend en outre un axe longitudinal et dans lequel une partie supplémentaire de la section centrale de l'ensemble doublure détachable s'étend au-delà du périmètre externe de la bande de fermeture de plaie le long de l'axe longitudinal de la bande de fermeture de plaie.

7. Dispositif selon l'une quelconque des revendications précédentes, dans lequel la section centrale de l'ensemble doublure détachable comprend au moins deux languettes situées au niveau de bords opposés de l'ensemble doublure détachable.

8. Dispositif selon l'une quelconque des revendications précédentes, dans lequel la section centrale est reliée au premier cadre et/ou au second cadre par une liaison frangible qui est conçue pour se rompre lors de la traction de la languette par rapport au premier et/ou au second cadre.

9. Dispositif selon l'une quelconque des revendications précédentes,
dans lequel chaque languette a une forme qui est sensiblement l'une des suivantes : triangulaire, elliptique, carrée, rectangulaire, pentagonale, et hexagonale.
